# EUROPÄISCHE PATENTSCHRIFT

(11) **EP 2 874 579 B1**
(45) Veröffentlichungstag und Bekanntmachung des Hinweises auf die Patenterteilung: **04.01.2017**
(21) Anmeldenummer: 13736809.8
(22) Anmeldetag: 26.06.2013
(51) Int. Cl.: A61F 5/01, A61F 5/058, A61F 5/37

(54) **SCHULTERABDUKTIONSORTHESE**
SHOULDER ABDUCTION ORTHOSIS
ORTHÈSE D'ABDUCTION D'ÉPAULE

(30) Priorität: 17.07.2012 DE 102012014541
(43) Veröffentlichungstag der Anmeldung: 27.05.2015
(73) Patentinhaber: Bauerfeind AG, 07937 Zeulenroda-Triebes (DE)
(72) Erfinder: BÄTZ, Ronny, 07937 Vogtl. Oberland OT Pöllwitz (DE); RÖBELT, Gerhard, 07950 Zeulenroda-Triebes (DE); STIER, Gerald, 07957 Langenwetzendorf (DE)
(74) Vertreter: Wohlfahrt, Jan Günther
(86) Internationale Anmeldenummer: PCT/EP2013/063443
(87) Internationale Veröffentlichungsnummer: WO 2014/012758

(56) Entgegenhaltungen:
- DE-T2- 60 209 459
- DE-T2- 69 616 629
- US-A- 4 241 731
- US-A- 5 538 499
- US-A1- 2004 010 213
- US-A1- 2012 101 419

## Beschreibung

Die Erfindung betrifft eine neuartige Schulterabduktionsorthese mit einer Beckenfassung, die eine Beckenschale, eine Bauchplatte und eine Rückenplatte umfasst, wobei die Bauchplatte und die Rückenplatte jeweils gelenkig mit der Beckenschale verbunden sind. Die erfindungsgemäße Schulterabduktionsorthese ermöglicht ein Tragen der Orthese ohne Schultergurt. Darüber hinaus lässt sich die Schulterabduktionsorthese leicht anziehen, insbesondere auch einhändig oder im Bett.

Orthesen sind therapeutische Hilfsmittel zur Stabilisierung oder Unterstützung der Bewegungsfunktion von Körperteilen, beispielsweise von Armen, Becken oder Wirbelsäule. Der Einsatz der Orthesen kann direkt posttraumatisch oder postoperativ oder konservativ erfolgen.

Schulterorthesen kommen zum Einsatz, wenn die Schulter ruhiggestellt oder in ihrer Bewegung gezielt eingeschränkt werden soll, beispielsweise nach Luxationen, operativen Eingriffen oder bei Lähmungen. Es gibt sie in verschiedenen Ausführungen, die je nach Indikation Anwendung finden. Eine dieser Ausführungen ist die Schulterabduktionsorthese.

Mithilfe einer Schulterabduktionsorthese wird ein Arm in einer vom Körper meist abgespreizten Position gelagert und ruhiggestellt. Schulterabduktionsorthesen finden zum Beispiel nach Schulteroperationen, Luxationen, Rotatorenmanschettenrupturen oder bei Schleimbeutelentzündungen in der Schulter Anwendung.

Schulterabduktionsorthesen gibt es in verschiedenen Ausführungen. Bei Modellen mit Luftkissen wird der Arm auf einem solchen gelagert. Das Kissen wird mit Gurten um die Taille, gesunde Schulter, Ober- und Unterarm am Körper fixiert. Solche sind beispielsweise in der DE 20 2007 003 117 U1 beschrieben.

Für eine stabilere Lagerung sorgen Schulterabduktionsorthesen mit Beckengurt samt Unterarm- und Handablage, wie sie aus der DE 33 90 447 T1 oder der US 2004/0153010 A1 bekannt sind. Die DE 696 16 629 T2 offenbart eine Schulterabduktionsorthese, die eine Beckenfassung und eine Armauflage umfasst, wobei die Beckenfassung eine Beckenschale, eine Bauchplatte und eine Rückenplatte umfasst, wobei die Armauflage über ein multiaxial bewegliches Gelenkelement mit der Beckenschale verbunden ist.

Damit die dort verwendeten Beckengurte durch den Druck des aufliegenden Arms nicht nach unten verrutschen werden diese Schulterabduktionsorthesen üblicherweise mit einem Schultergurt versehen, der über die Gegenschulter geführt ist. Solche Schultergurte verringert jedoch den Tragekomfort und kann darüber hinaus zu Fehlbelastungen der Halswirbelsäule führen. Weiterhin ist die Gurtführung im Brustbereich insbesondere bei weiblichen Patienten problematisch.

Insgesamt sind die zurzeit verwendeten Schulterabduktionsorthesen konstruktiv aufwendig, weißen einen geringen Tragekomfort auf und/oder sind, insbesondere für einen Patienten mit verletztem Arm oder verletzter Schulter, schwer anzuziehen. Soweit bekannte Systeme in der Vergangenheit ohne Schultergurt auskamen, so war ein wesentlicher Nachteil, dass diese insbesondere bei schwereren Armen von Patienten wegen des Gewichts verrutschten. Dies wurde zu verhindern versucht, indem der Bauchgurt sehr stark gespannt wurde. Damit wurden die darunter liegenden Gewebeschichten sehr stark komprimiert, was häufig als schmerzhaft empfunden wurde. Wegen der starken Komprimierung war eine Sitzposition mit der Orthese nur kurzzeitig einnehmbar.

Die Erfindung hat sich die Aufgabe gestellt, Schulterabduktionsorthesen weiterzuentwickeln und zu verbessern, um die bekannten Nachteile zu vermeiden. Das der Erfindung zugrunde liegende technische Problem liegt in der Bereitstellung einer verbesserten Schulterabduktionsorthese, die insbesondere bequem zu tragen ist und leicht durch einen Patienten, der in der Beweglichkeit eines Arms beschränkt ist, angezogen werden kann und/oder einem in einem Bett liegenden Patienten angezogen werden kann. Ein weiteres der Erfindung zugrunde liegende technische Problem liegt in der Bereitstellung einer verbesserten Schulterabduktionsorthese, die ohne Schultergurt getragen werden kann und dennoch bequem ist und nicht verrutscht. Dabei soll die Grundfunktion der Schulterabduktionsorthese beibehalten bleiben und möglichst flexibel sein.

Das technische Problem wird vollständig gelöst durch eine Schulterabduktionsorthese nach Anspruch 1.

Das technische Problem wird insbesondere gelöst durch eine Schulterabduktionsorthese, umfassend eine Beckenfassung, und eine Armauflage, wobei die Beckenfassung eine Beckenschale, eine Bauchplatte und eine Rückenplatte umfasst, wobei die Bauchplatte und die Rückenplatte jeweils gelenkig mit der Beckenschale verbunden sind und wobei die Armauflage über ein multiaxial bewegliches Gelenkelement mit der Beckenschale verbunden ist, wobei die Bauchplatte und die Rückenplatte jeweils mindestens 10 cm breit sind, wobei die Beckenfassung mindestens ein Gurtsystem zum Verbinden der Bauchplatte mit der Rückenplatte aufweist, wobei das mindestens eine Gurtsystem mit der Bauchplatte und/oder in die Rückenplatte reversibel gekoppelt werden kann und wobei das mindestens eine gekoppelte Gurtsystem nachgespannt werden kann.

Die Verwendung einer Beckenfassung mit einer an einer Beckenplatte gelenkig verbundenen Bauchplatte und einer an einer Beckenplatte gelenkig verbundenen Rückenplatte, die ein Gurtsystem zum Verbinden der Bauchplatte mit der Rückenplatte aufweisen, das mit der Bauchplatte und/oder in die Rückenplatte reversibel gekoppelt werden kann und wobei das mindestens eine gekoppelte Gurtsystem nachgespannt werden kann erlaubt in überraschender Weise, dass die Schulterabduktionsorthese leicht angezogen werden kann. Ein Patient, der in der Beweglichkeit eines Arms beschränkt ist, kann beispielsweise die Schulterabduktionsorthese in eine Sitzmöglichkeit mit Armlehne oder auf einen Tisch stellen und einarmig die Bauchplatte vor seinen Körper und die Rückenplatte Hinter seinen Körper schieben und ebenfalls dann einarmig die Bauchplatte und die Rückenplatte mit dem Gurtsystem koppeln und das Gurtsystem nachspannen, und dann den verletzten Arm in die Armauflage einlegen. Er kann also ohne fremde Hilfe die Schulterabduktionsorthese anziehen, obwohl er nur einen Arm zur Verfügung hat. Auch kann durch die Ausgestaltung der Körperumfassung mit Bauchplatte und Rückenplatte die Schulterabduktionsorthese einem auf dem Rücken im Bett liegenden Patienten leicht angezogen werden, da durch die Steifigkeit der Rückenplatte diese leicht unter dem Patienten hindurch geschoben bzw. gezogen werden kann und das Gurtsystem dann gekoppelt und nachgespannt werden kann.

Die Bauchplatte und die Rückenplatte zeichnen sich durch eine höhere Steifigkeit gegenüber einem reinen Gurt aus.

Darüber hinaus wird dieses Anziehen auch durch die Breite der Bauchplatte und der Rückenplatte von mindestens 10 cm erleichtert, da dies die Steifigkeit der Bauchplatte und der Rückenplatte erhöht. Zusätzlich trägt diese Breite auch zu einem sicheren und dennoch bequemen Sitz der Schulterabduktionsorthese bei, insbesondere wenn kein Schultergurt verwendet wird.

Auch die gelenkige Verbindung der Bauchplatte und der Rückenplatte mit der Beckenschale trägt zu einer verbesserten Einstellbarkeit, bei, da durch die Winkeleinstellung ein optimales anpassen an die jeweilige Beckenform möglich ist. Dadurch wird wiederum die Tragestabilität, auch bei einer Verwendung ohne Schultergurt, erhöht.

In einer bevorzugten Ausführungsform ist die Bauchplatte und die Rückenplatte jeweils mindestens 20 cm, insbesondere jeweils mindestens 30 cm lang, besonders bevorzugt jeweils mindestens 40 cm lang.

In einer bevorzugten Ausführungsform reicht die Bauchplatte in ihrer Länge mindestens bis zur Mitte des Bauches. In einer bevorzugten Ausführungsform reicht die Rückenplatte in ihrer Länge mindestens bis zur Mitte des Rückens. In einer bevorzugten Ausführungsform reicht die Bauchplatte in ihrer Länge mindestens bis zur Mitte des Bauches, wobei die Rückenplatte in ihrer Länge mindestens bis zur Mitte des Rückens reicht.

In einer bevorzugten Ausführungsform reicht die Bauchplatte in ihrer Länge über den Bauch. In einer bevorzugten Ausführungsform reicht die Rückenplatte in ihrer Länge über den Rücken. In einer bevorzugten Ausführungsform reicht die Bauchplatte in ihrer Länge über den Bauch, wobei die Rückenplatte in ihrer Länge über den Rücken reicht.

Bauchplatte und Rückenplatte können gleich oder verschieden lang sein.

Bauch und/oder Rückenplatte können so ausgestaltet sein, dass sie beispielsweise durch Abschneiden einer bestimmten Länge des jeweiligen Endstücks auf die individuell passende länge gekürzt werden.

In einer bevorzugten Ausführungsform sind die Bauchplatte und die Rückenplatte so lang, dass sie im angezogenen Zustand so liegen, dass ihre beiden Enden an der der Beckenplatte gegenüber liegenden Seite des Patientenkörpers anliegen. Dies ermöglicht, dass das Gurtsystem mit der Bauchplatte im bereich des Bauches und mit der rückenplatte im Bereich des Rückens gekoppelt werden kann und verstärkt den sicheren Halt der Schulterabduktionsorthese.

Bevorzugt haben die Enden der Bauchplatte und der Rückenplatte im angezogenen Zustand der Schulterabduktionsorthese einen Abstand von höchstens etwa 60 cm, bevorzugt höchstens 30 cm insbesondere höchstens 10 cm, oder stoßen direkt aneinander. Dies erhöht die Tragestabilität und auch den Tragekomfort, insbesondere wenn die Bauchplatte und die Rückenplatte auf den Innenflächen gepolstert sind. Insbesondere wird dadurch bevorzugt verhindert dass das Gurtsystem direkt auf den Körper, insbesondere auf die Rumpfseite des Patienten drückt. Jedoch kann bei einem großen Brustumfang auch bei Bedarf ein größerer Abstand zwischen den Enden der Bauchplatte und der Rückenplatte vorgesehen sein.

Bevorzugt sind die Innenflächen der Bauchplatte und/oder der Rückenplatte gepolstert. Bevorzugt ist, insbesondere auch, die Innenseite der Beckenschale gepolstert. Ein Fachmann kennt geeignete Polsterungsmöglichkeiten und Polsterungsmaterialien.

In einer bevorzugten Ausführungsform sind die Bauchplatte und die Rückenplatte biegbar ausgebildet. Die Bauchplatte und die Rückenplatte sind in dieser Ausführungsform also steifer als Gurte, aber dennoch biegbar, insbesondere über ihre Länge. Eine solche Biegbarkeit verbessert den Tragekomfort, da sich die Bauchplatte und die Rückenplatte der Körperform zumindest größtenteils anpassen können.

Die erfindungsgemäße Schulterabduktionsorthese zeichnet sich nicht nur durch eine einfache Anziehbarkeit aus, sondern auch durch einen hohen Tragekomfort, zum Beispiel auch in einer Sitzposition des Patienten.

Ein Fachmann kennt geeignete Materialien, insbesondere Kunststoffe, aus denen solche Bauchplatten und Rückenplatten hergestellt werden können.

In einer bevorzugten Ausführungsform sind die Bauchplatte und die Rückenplatte baugleich. In einer bevorzugten Ausführungsform sind die Bauchplatte und die Rückenplatte spiegelsymmetrisch. Dadurch kann erreicht werden, dass Schulterabduktionsorthese ohne weiteres für den rechten Arm oder für den linken Arm verwendet werden kann, da eine Platte entweder als Bauchplatte oder als Rückenplatte und die andere Platte entweder als Rückenplatte oder als Bauchplatte verwendet werden kann.

In einer alternativen Ausführungsform kann auch vorgesehen sein, dass die Bauchplatte und die Rückenplatte nicht baugleich sind. Insbesondere für Patienten mit erhöhter Körperfülle kann dabei bevorzugt vorgesehen sein, dass die Bauchplatte an der oberen Kannte eine konkave Wölbung aufweist, dass die Bauchplatte dort also im mittleren Bereich verjüngt ist, so dass der Bauch insbesondere in einer Sitzposition nicht so sehr eingeengt wird.

In einer bevorzugten Ausführungsform ist die Schulterabduktionsorthese eine Schultergurt-freie Schulterabduktionsorthese. Bevorzugt weist die Schulterabduktionsorthese also keinen Schultergurt auf. Bevorzugt kann daher zur Einsparung von Materialien, Gewicht und zusätzlichen Elementen vorgesehen sein, dass die Schulterabduktionsorthese keine Elemente zur Befestigung eines Schultergurts aufweist. Es kann natürlich auch vorgesehen sein, dass die Schulterabduktionsorthese wahlweise mit einem Schultergurt aufgerüstet werden kann.

Neben den oben genannten positiven Effekten einer Schultergurt-freie Schulterabduktionsorthese, also verbesserter Tragekomfort und Verhinderung von Fehlbelastung der Halswirbelsäule, kann eine solche Schulterabduktionsorthese auch leichter angezogen werden, da ein Patient, der in der Beweglichkeit eines Arms beschränkt ist, nicht den Schultergurt über die Schulter ziehen und anschließend befestigen muss.

Ein Schultergurt wird im Stand der Technik üblicherweise verwendet, damit die Schulterabduktionsorthese sicher am Rumpf des Patienten hält und nicht verrutscht, insbesondere durch den Druck des aufliegenden Arms und der Bewegung des Patienten, beispielsweise Lauf- oder Drehbewegung. Durch einen Schultergurt wird die Schulterabduktionsorthese oben gehalten und kann nicht nach unten rutschen.

Es hat sich überraschender Weise gezeigt, dass bei einer erfindungsgemäßen Schulterabduktionsorthese auf einen Schultergurt verzichtet werden kann, ohne dass die Schulterabduktionsorthese beim Tragen verrutscht, insbesondere nach unten rutscht. Dies wird insbesondere durch die Verwendung einer Beckenschale, die auf dem Beckenkamm aufliegen kann, in Kombination mit den mindestens 10 cm breiten Bauchplatten und Rückenplatten, die gelenkig mit der Beckenschale verbunden sind, erreicht. Weiter verstärkt kann dieser Effekt werden durch bevorzugte Ausführungsformen, bei denen die Beckenschale eine Pelotte aufweist und/oder bei denen das mindestens eine Gurtsystem mit der Bauchplatte und/oder in die Rückenplatte über mindestens zwei Hintergriffelemente reversibel gekoppelt werden kann. Der sichere Halt ohne Schultergurt kann bei der erfindungsgemäßen Schulterabduktionsorthese überraschender Weise auch bei Patienten mit einem großen Bauchumfang und bei Patienten mit schweren Armen erreicht werden. Auch bei diesen Patienten kann mit der erfindungsgemäßen Schulterabduktionsorthese ein Herunterrutschen der Schulterabduktionsorthese verhindert werden, wobei dabei in vorteilhafter Weise das Gurtsystem nicht so stark festgezogen werden muss, dass das Tragen der Schulterabduktionsorthese sehr unangenehm wird.

Das Zusammenspiel dieser Ausführungsformen führt in synergistischer Weise zu einem besonders guten Halt der Schulterabduktionsorthese, insbesondere auch dadurch dass sich die Schulterabduktionsorthese besonders gut der Anatomie des jeweiligen Patienten anpasst, wobei die Schulterabduktionsorthese aber dennoch eine konfektionierte Schulterabduktionsorthese sein kann.

Bevorzugt ist die Schulterabduktionsorthese eine konfektionierte Schulterabduktionsorthese, die zumindest in ihrem Grundaufbau nicht an einen spezifischen Patienten angepasst ist. Insbesondere muss die Schulterabduktionsorthese bevorzugt nicht durch thermische Verformung an die Anatomie eines spezifischen Patienten angepasst werden.

Natürlich kann die Schulterabduktionsorthese der Aufnahme des rechten oder des linken Arms dienen. Dementsprechend kann die Beckenschale und damit die Armauflage entweder rechts oder links angebracht sein.

In einer bevorzugten Ausführungsform kann das mindestens eine gekoppelte Gurtsystem über mindestens einen Klettverschluss nachgespannt werden. Dies ermöglicht ein einfaches Nachspannen auch mit nur einer Hand.

In einer bevorzugten Ausführungsform kann das mindestens eine Gurtsystem mit der Bauchplatte und/oder in die Rückenplatte über mindestens zwei Hintergriffelemente reversibel gekoppelt werden. In einer bevorzugten Ausführungsform kann das mindestens eine Gurtsystem mit der Bauchplatte und/oder in die Rückenplatte über zwei Hintergriffelemente reversibel gekoppelt werden. Eine solche Ausgestaltungsform ermöglicht einen sicheren Halt des reversibel gekoppelten Gurtsystems in der Bauchplatte und der Rückenplatte beim Tragen der Schulterabduktionsorthese durch einen Patienten trotz der auftretenden Körperbewegungen.

In einer bevorzugten Ausführungsform sind die Bauchplatte und die Rückenplatte jeweils mindestens 15 cm breit.

In einer bevorzugten Ausführungsform sind die Bauchplatte und die Rückenplatte jeweils mindestens 10 cm breit. In einer sehr bevorzugten Ausführungsform sind die Bauchplatte und die Rückenplatte jeweils mindestens 12 cm breit. Die die Bauchplatte und die Rückenplatte können jeweils beispielsweise etwa 14 cm breit sein. In einer bevorzugten Ausführungsform sind die Bauchplatte und die Rückenplatte jeweils höchstens 25 cm breit. In einer sehr bevorzugten Ausführungsform sind die Bauchplatte und die Rückenplatte jeweils höchstens 18 cm breit.

In einer bevorzugten Ausführungsform ist das mindestens eine Gurtsystem mindestens 10 cm breit. In einer sehr bevorzugten Ausführungsform ist das mindestens eine Gurtsystem mindestens 12 cm breit. Das Gurtsystem kann beispielsweise etwa 14 cm breit sein. In einer bevorzugten Ausführungsform ist das mindestens eine Gurtsystem höchstens 25 cm breit. In einer sehr bevorzugten Ausführungsform ist das mindestens eine Gurtsystem höchstens 18 cm breit.

In einer bevorzugten Ausführungsform weist die Beckenschale mindestens eine Pelotte zur Rutschhemmung auf. In einer bevorzugten Ausführungsform weist die Beckenschale eine Pelotte zur Rutschhemmung auf. In einer bevorzugten Ausführungsform weist die Beckenschale im mittleren Bereich der Beckenschale eine Pelotte auf. In einer bevorzugten Ausführungsform weist die Beckenschale im mittleren Höhenbereich der Beckenschale eine Pelotte auf. In einer bevorzugten Ausführungsform ist also die Pelotte im mittleren Bereich der Beckenschalenhöhe an die Beckenschale angebracht.

Wie bereits erwähnt verbessert eine solche Pelotte nochmals das Verhindern eines Verrutschens, insbesondere nach unten, der Schulterabduktionsorthese beim Tragen durch den Patienten.

In einer bevorzugten Ausführungsform ist die Beckenschale mindestens 10 cm, insbesondere mindestens 15 cm, bevorzugt mindestens 20 cm hoch, besonders bevorzugt mindestens 25 cm hoch. Die Beckenschale kann beispielsweise etwa 27 cm hoch sein. In einer bevorzugten Ausführungsform ist die Beckenschale höchstens 45 cm, insbesondere höchstens 40 cm, bevorzugt höchstens 35 cm hoch, besonders bevorzugt höchstens 30 cm hoch.

In einer bevorzugten Ausführungsform ist die Beckenschale so ausgestaltet, dass sie mit ihrem unteren Teil am Ende des Beckens aufliegt.

In einer bevorzugten Ausführungsform ist die gelenkige Verbindung der Bauchplatte und der Rückenplatte mit der Beckenschale fixierbar.

In einer bevorzugten Ausführungsform sind die Bauchplatte und Rückenplatte mit der Beckenschale jeweils über ein Drehgelenk verbunden. In einer bevorzugten Ausführungsform sind die Bauchplatte und Rückenplatte mit der Beckenschale jeweils über ein scheibenförmiges Drehgelenk verbunden.

In einer bevorzugten Ausführungsform sind die Bauchplatte und Rückenplatte mit der Beckenschale jeweils über ein scheibenförmiges Drehgelenk fixierbar verbunden.

Erfindungsgemäß weist die Schulterabduktionsorthese eine Armauflage auf. Erfindungsgemäß ist die Armauflage mit der Beckenschale verbunden, wobei sie auch reversibel verbunden sein kann. Bevorzugt erfolgt die Verbindung mit der Beckenschale über ein multiaxial bewegliches Gelenkelement. Einem Fachmann sind geeignete multiaxial bewegliche Gelenkelemente bekannt. Diese ermöglichen bevorzugt auch eine stufenweise Freigabe der Bewegungsmöglichkeit des Arms und/oder der Schulter während des Therapieverlaufs. Auch können die Gelenke der Armauflage und das multiaxial bewegliche Gelenkelement mit einer Gegenkraft beauflagt werden, beispielsweise einer Federkraft, um dem Patienten Reha-Maßnahmen nach Anweisung des Betreuungspersonals auch zu Hause zu ermöglichen. Die Gegenkräfte sind bevorzugt in ihrer Stärke individuell einstellbar.

In einer bevorzugten Ausführungsform ist das multiaxial bewegliche Gelenkelement oberhalb der Pelotte mit der Beckenschale verbunden. In einer bevorzugten Ausführungsform ist das multiaxial bewegliche Gelenkelement am oberen Ende oder im Bereich des oberen Endes mit der Beckenschale verbunden. Bevorzugt ist das multiaxial bewegliche Gelenkelement höchstens 5 cm vom oberen Ende der Beckenschale entfernt.

Die Armauflage der Schulterabduktionsorthese kann verschiedenartig ausgebildet sein. Ein Fachmann kennt geeignete Ausführungsformen einer Armauflage.

Die Armauflage der Schulterabduktionsorthese ist bevorzugt so ausgebildet, dass der aufliegende Arm zwar abgespreizt werden kann, aber nicht muss. Bevorzugt ist der Arm mindestens 10° , insbesondere mindestens 15° abgespreizt.

In einer bevorzugten Ausführungsform weist die Schulterabduktionsorthese eine Oberarmauflage und eine Unterarmauflage auf.

Weiter bevorzugte Ausführungsformen der Erfindung ergeben sich aus den Unteransprüchen und den Nebenansprüchen.

Die Erfindung wird durch die nachfolgenden Figuren und Figurenbeschreibungen näher illustriert, ohne dass diese beschränkend zu verstehen wären.

Es zeigen
- Figur 1: eine erfindungsgemäße Schulterabduktionsorthese ohne Gurtsystem;
- Figur 2: die Beckenfassung einer erfindungsgemäßen Schulterabduktionsorthese;
- Figur 3: eine alternative Ausführungsform einer erfindungsgemäßen Schulterabduktionsorthese ohne Gurtsystem von oben;
- Figur 4: eine Vorderansicht einer angezogenen erfindungsgemäßen Schulterabduktionsorthese mit Gurtsystem;
- Figur 5: eine Seitenansicht der angezogenen erfindungsgemäßen Schulterabduktionsorthese mit Gurtsystem von Figur 4;
- Figur 6: eine Rückansicht der angezogenen erfindungsgemäßen Schulterabduktionsorthese mit Gurtsystem von Figur 4.

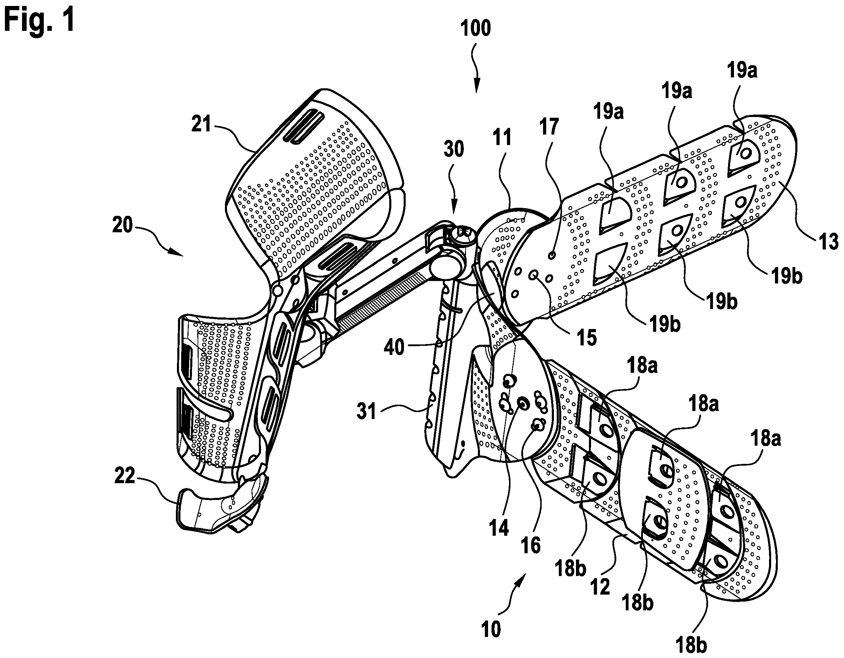

Figur 1 zeigt eine erfindungsgemäße Schulterabduktionsorthese (100), umfassend eine Beckenfassung (10), und eine Armauflage (20). Die gezeigte Schulterabduktionsorthese (100) dient der Abstützung und Fixierung des rechten Arms bzw. der rechten Schulter eines Patienten. Die Beckenfassung (10) umfasst eine Beckenschale (11), eine Bauchplatte (12) und eine Rückenplatte (13). Im vorliegenden Fall sind die Bauchplatte (12) und die Rückenplatte (13) baugleich. Beckenschale (11), Bauchplatte (12) und Rückenplatte (13) weisen eine Vielzahl von Perforationen auf, die zu einem besseren Luftaustausch und zu einer Gewichtsreduktion beitragen.

Die Armauflage (20) weist einen ersten Abschnitt (21) und einen zweiten Abschnitt (22) auf. Der erste Abschnitt (21) dient der Aufnahme des Oberarms und des Unterarms, der zweite Abschnitt (22) dient der Aufnahme des Handgelenks. Der Arm kann auf der Armauflage (20) beispielsweise durch Klettverschlüsse oder Gurte fixiert werden. Spezielle Ausführungsformen einer Armauflage sind dem Fachmann bekannt, so dass er die gezeigte Ausführungsform ohne weiteres modifizieren kann.

Die Armauflage (20) ist über ein multiaxial bewegliches Gelenkelement (30) mit der Beckenschale (10) verbunden. Dadurch kann ein gewünschter Winkel eingestellt werden, mit dem der Arm vom Oberkörper abgespreizt ist. Dabei kann wahlweise auch ein sehr kleiner Winkel, zum Beispiel 15° oder sogar weniger gewählt werden. Das Gelenkelement (30) kann zum Beispiel drehbar in das Aufnahmeelement (31) eingeschoben sein, wobei natürlich eine Fixierbarkeit des Gelenkelements (30) im Aufnahmeelement vorgesehen ist.

Die Bauchplatte (12) und die Rückenplatte (13) sind jeweils über einen Drehpunkt (14, 15) gelenkig mit der Beckenschale (11) verbunden. Das Drehgelenk umfasst nicht nur den Drehpunkt (14, 15) sondern ist mit weiteren Verbindungselementen (16, 17) scheibenförmig ausgebildet. Die Drehgelenke sind fixierbar, so dass die Position der Bauchplatte (12) sowie der Rückenplatte (13) zur Beckenschale (11) eingestellt und dann fixiert werden kann.

Die Bauchplatte (12) und die Rückenplatte (13) sind vorliegend etwa 14 cm breit und aus Kunststoff. Dies gibt der Beckenfassung (10) einen guten Halt. Auch die Beckenschale (11) ist aus Kunststoff.

Die Beckenschale (11) weist eine Pelotte (40) zur Rutschhemmung auf. Diese ist im mittleren Bereich der Beckenschale (11) positioniert. Die Pelotte (40) kann auf dem Beckenkamm des Patienten aufliegen und so ebenfalls sehr gut das Herunterrutschen der Beckenfassung (10) und somit der Schulterabduktionsorthese (100) verhindern.

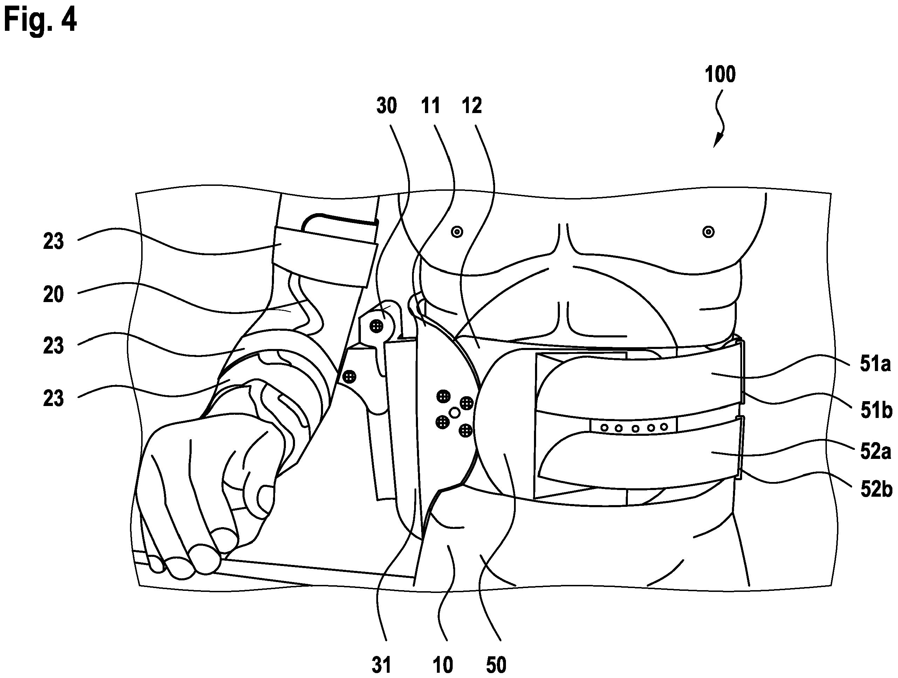

Nicht gezeigt ist ein Gurtsystem zum Verbinden der Bauchplatte (12) mit der Rückenplatte (13). Ein solches Gurtsystem kann vorliegend mit der Bauchplatte (12) und mit der Rückenplatte (13) reversibel gekoppelt werden. Das Gurtsystem kann über ein jeweils übereinander liegendes Hintergriffelementpaar (18a, 18b) mit der Bauchplatte (12) und über ein jeweils übereinander liegendes Hintergriffelementpaar (19a, 19b) mit der Rückenplatte (13) reversibel gekoppelt werden. Dabei kann je nach Bauchumfang des Patienten eines der drei Hintergriffelementpaare (18a, 18b) sowie eines der drei Hintergriffelementpaare (19a, 19b) gewählt werden, so dass der Sitz der Beckenschale (10) gut an den Bauchumfang des Patienten angepasst werden kann. Darüber hinaus kann das Gurtsystem bevorzugt noch nachgespannt werden, zum Beispiel über zwei Klettverschlüsse, wie in den Figuren 4 bis 6 gezeigt.

Natürlich kann die Beckenfassung (10) der Schulterabduktionsorthese (100) auch zur Abstützung und Fixierung des linken Arms bzw. der linken Schulter eines Patienten verwendet werden, wobei dann die Schulterabduktionsorthese (100) einfach gedreht werden kann und die Bauchplatte (12) als Rückenplatte und die Rückenplatte (13) als Bauchplatte verwendet wird. Es muss dann nur eine Armauflage (20) verwendet werden, die für die Aufnahme eines linken Arms ausgestaltet ist. Diese kann dann zum Beispiel einfach in das Aufnahmeelement (31) eingeschoben werden oder am Gelenkelement (30) anstelle der gezeigten Armauflage (20) befestigt werden.

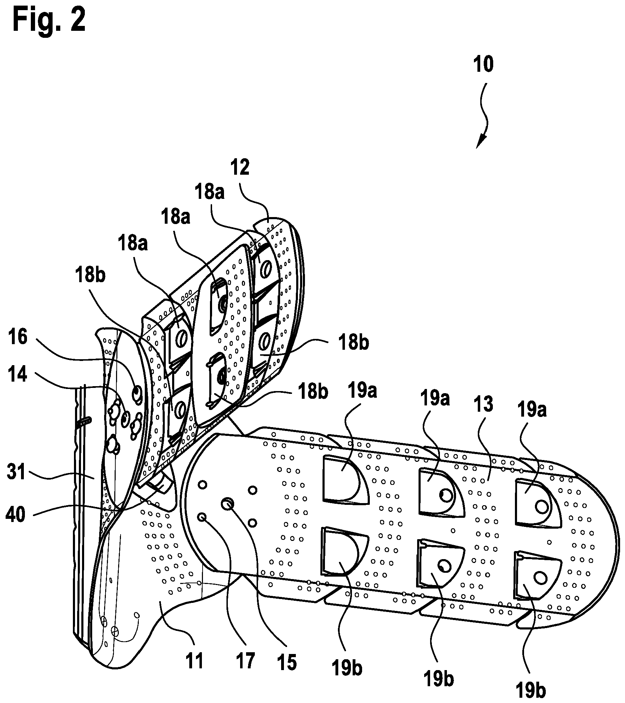

Figur 2 zeigt die Beckenfassung (10) von Figur 1 aus einer anderen Perspektive, wobei die Armauflage und das multiaxial bewegliches Gelenkelement zur besseren Übersicht weggelassen wurde. Zu sehen sind wieder die Beckenschale (11) mit Pelotte (40) und Aufnahmeelement (31), die Bauchplatte (12) mit den Hintergriffelementen (18a, 18b) und die Rückenplatte (13) mit den Hintergriffelementen (19a, 19b). Auch die beiden fixierbaren scheibenförmigen Drehgelenke (14, 15, 16, 17) sind zu sehen.

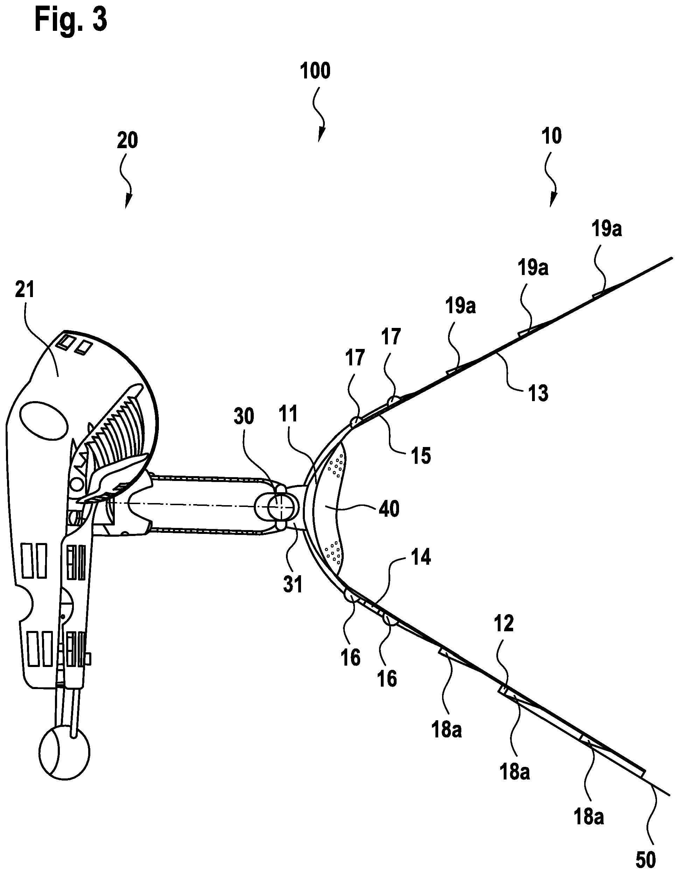

Figur 3 zeigt eine alternative Ausführungsform der erfindungsgemäßen Schulterabduktionsorthese (100) von oben. Nicht gezeigt ist wieder das Gurtsystem zum Verbinden der Bauchplatte (12) mit der Rückenplatte (13). Zu sehen sind wieder eine Beckenfassung (10) mit Beckenschale (11) mit Pelotte (40) und Aufnahmeelement (31), die Bauchplatte (12) mit den Hintergriffelementen (18a, 18b) und die Rückenplatte (13) mit den Hintergriffelementen (19a, 19b). Auch die beiden fixierbaren scheibenförmigen Drehgelenke (14, 15, 16, 17) sind zu sehen.

Die Armauflage (20) mit dem Trageelement (21) ist über ein multiaxial bewegliches Gelenkelement (30) mit der Beckenfassung (10) verbunden. Die Armauflage (20) ist etwas anders gestaltet als die Armauflage (20) der Figuren 1 und 2.

Figur 4 zeigt eine erfindungsgemäße Schulterabduktionsorthese (100) an einem Patienten von vorne. Gezeigt ist dabei insbesondere auch das Gurtsystem (50) zum reversiblen Koppeln der Bauchplatte (12) und der nicht sichtbaren Rückenplatte.

Die Schulterabduktionsorthese (100) weist wieder eine Beckenfassung (10) mit Beckenschale (11) mit Aufnahmeelement (31), Bauchplatte (12) und Rückenplatte sowie eine Armauflage (20), das über ein multiaxial bewegliches Gelenkelement (30) mit der Beckenfassung (10) verbunden ist, auf. Der Arm des Patienten ist auf die Armauflage (20) aufgelegt und mit Klettverschlüssen (23) an dieser fixiert.

In ein Paar von Hintergriffelementen der Bauchplatte (12) ist das Gurtsystem (50) eingehakt. Die paarige Ausführungsform der Hintergriffelemente führt zu einem besonders guten Halt der Beckenfassung (10), auch wenn sich der Patient bewegt. Das Gurtsystem (50) weist zwei Klettverschlusslaschen (51 a, 52a) auf, die über zwei Umlenkstege (51 b, 52b) geführt sind. Die Klettverschlusslaschen (51 a, 52a) ermöglichen das Nachspannen des Gurtsystems (50) nachdem die Schulterabduktionsorthese (100) vom Patienten angelegt wurde und das Gurtsystem (50) in die Bauchplatte (12) eingehakt wurde, so dass die Schulterabduktionsorthese (100) fest und sicher sitzt und nicht verrutscht. Das Gurtsystem (50) kann dabei in vorteilhafter Weise von dem Patienten selbst einhändig mit der linken Hand in die Bauchplatte (12) eingehakt werden. Auch das Nachspannen durch die Klettverschlusslaschen (51 a, 52a) kann in vorteilhafter Weise durch den Patienten selbst einhändig mit der linken Hand erfolgen.

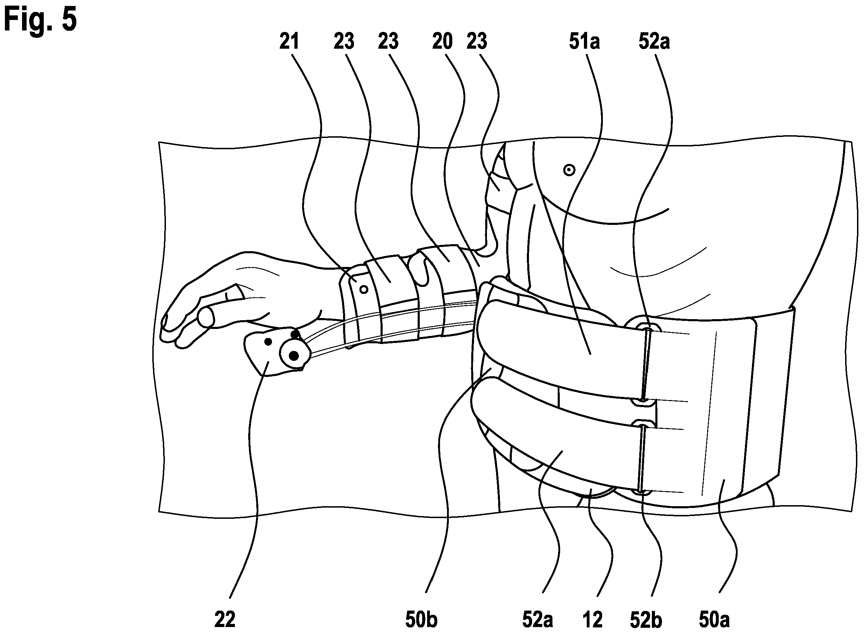

Figur 5 zeigt die erfindungsgemäße Schulterabduktionsorthese (100) von Figur 4 an einem Patienten von der Seite. Gezeigt ist dabei insbesondere wieder das Gurtsystem (50) zum reversiblen Koppeln der Bauchplatte (12) und der nicht sichtbaren Rückenplatte.

In ein Paar von Hintergriffelementen der Bauchplatte (12) ist das vordere Teilelement (50b) des Gurtsystems (50) eingehakt. Das hintere Teilelement (50a) des Gurtsystems (50) ist in der Figur nicht sichtbar in die Rückenplatte eingehakt. Das Gurtsystem (50) weist zwei Klettverschlusslaschen (51 a, 52a) auf, die über zwei Umlenkstege (51 b, 52b) geführt sind. Die Klettverschlusslaschen (51 a, 52a) sind an dem vorderen Teilelement (50b) des Gurtsystems (50) befestigt und die Umlenkstege (51 b, 52b) sind an dem hinteren Teilelement (50a) des Gurtsystems (50) befestigt. So wird wieder das Nachspannen des Gurtsystems (50) ermöglicht.

Der Arm des Patienten ist auf wieder auf die Armauflage (20) mit dem oberen Element (21) aufgelegt und mit Klettverschlüssen (23) an die Armauflage (20) fixiert.

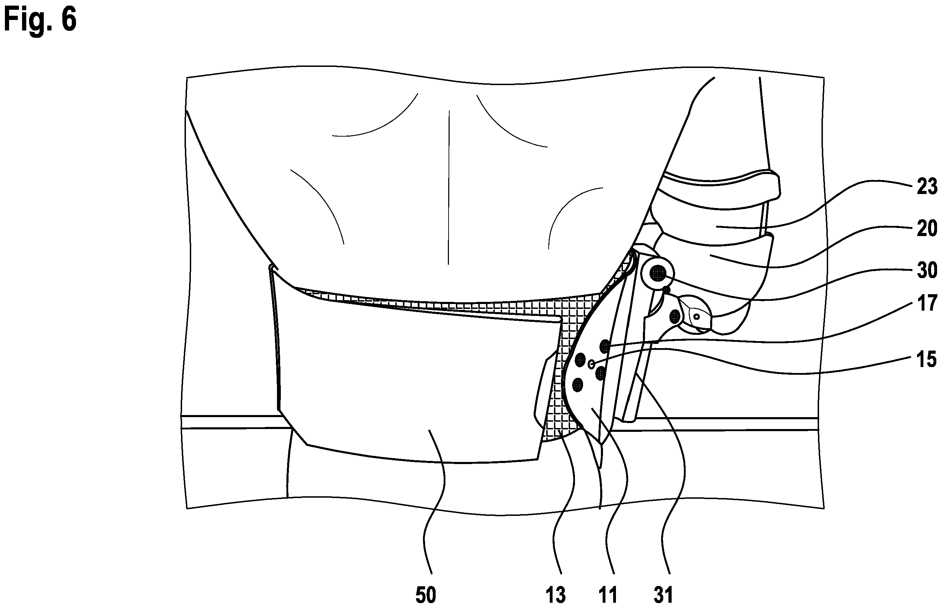

Figur 6 zeigt die erfindungsgemäße Schulterabduktionsorthese (100) von Figur 4 an einem Patienten von hinten. Gezeigt ist dabei insbesondere wieder das Gurtsystem (50) zum reversiblen Koppeln der nicht sichtbaren Bauchplatte und der Rückenplatte (13).

Die Schulterabduktionsorthese (100) weist wieder eine Beckenfassung mit Beckenschale (11) mit Aufnahmeelement (31), Bauchplatte und Rückenplatte (13) sowie eine Armauflage (20), das über ein multiaxial bewegliches Gelenkelement (30) mit der Beckenfassung (10) verbunden ist, auf. Auch die das fixierbare scheibenförmige Drehgelenk (15, 17) zwischen Beckenschale (11) und Rückenplatte (13) ist zu sehen. Der Arm des Patienten ist auf die Armauflage (20) aufgelegt und mit Klettverschlüssen (23) an dieser fixiert.

In ein Paar von Hintergriffelementen der Rückenplatte (13) ist wieder das Gurtsystem (50) eingehakt.

## Patentansprüche

1. Schulterabduktionsorthese (100), umfassend eine Beckenfassung (10), und eine Armauflage (20), wobei die Beckenfassung (10) eine Beckenschale (11), eine Bauchplatte (12) und eine Rückenplatte (13) umfasst, wobei die Armauflage (20) über ein multiaxial bewegliches Gelenkelement (30) mit der Beckenschale (10) verbunden ist, und wobei die Bauchplatte (12) und die Rückenplatte (13) jeweils mindestens 10 cm breit sind,
wobei die Beckenfassung (10) mindestens ein Gurtsystem (50) zum Verbinden der Bauchplatte (12) mit der Rückenplatte (13) aufweist, wobei das mindestens eine Gurtsystem (50) mit der Bauchplatte (12) und/oder mit der Rückenplatte (13) reversibel gekoppelt werden kann und wobei das mindestens eine gekoppelte Gurtsystem (50) nachgespannt werden kann **dadurch gekennzeichnet,**
**dass** die Bauchplatte (12) und die Rückenplatte (13) jeweils gelenkig mit der Beckenschale (11) verbunden sind.

2. Schulterabduktionsorthese nach Anspruch 1, wobei die Bauchplatte (12) in ihrer Länge mindestens bis zur Mitte des Bauches reicht und wobei die Rückenplatte (13) in ihrer Länge mindestens bis zur Mitte des Rückens reicht.

3. Schulterabduktionsorthese nach einem der vorhergehenden Ansprüche, wobei die Bauchplatte (12) und die Rückenplatte (13) biegbar ausgebildet sind.

4. Schulterabduktionsorthese nach einem der vorhergehenden Ansprüche, wobei die Schulterabduktionsorthese (100) eine Schultergurt-freie Schulterabduktionsorthese ist.

5. Schulterabduktionsorthese nach einem der vorhergehenden Ansprüche, wobei das mindestens eine gekoppelte Gurtsystem (50) über mindestens einen Klettverschluss (51, 52) nachgespannt werden kann.

6. Schulterabduktionsorthese nach einem der vorhergehenden Ansprüche, wobei das mindestens eine Gurtsystem (50) mit der Bauchplatte (12) und/oder mit der Rückenplatte (13) über jeweils zwei Hintergriffelemente (18a, 18b, 19a, 19b) reversibel gekoppelt werden kann.

7. Schulterabduktionsorthese nach einem der vorhergehenden Ansprüche, wobei das mindestens eine Gurtsystem (50) mindestens 10 cm breit ist.

8. Schulterabduktionsorthese nach einem der vorhergehenden Ansprüche, wobei die Beckenschale (11) eine Pelotte (40) zur Rutschhemmung aufweist.

9. Schulterabduktionsorthese nach einem der vorhergehenden Ansprüche, wobei die Bauchplatte (12) und Rückenplatte (13) mit der Beckenschale (11) jeweils über ein scheibenförmiges Drehgelenk (14, 15, 16, 17) fixierbar verbunden sind.

10. Schulterabduktionsorthese nach einem der vorhergehenden Ansprüche, wobei die Schulterabduktionsorthese (100) eine Oberarmauflage (21) und eine Unterarmauflage (21, 22) aufweist.

## Claims

1. Shoulder abduction orthosis (100), comprising a pelvic harness (10) and an arm support (20), wherein the pelvic harness (10) comprises a pelvic shell (11), an abdominal plate (12) and a back plate (13), wherein the arm support (20) is connected to the pelvic shell (10) by means of a multiaxially movable articulation element (30), and wherein the abdominal plate (12) and the back plate (13) each have a width of at least 10 cm,
wherein the pelvic harness (10) has at least one belt system (50) for connecting the abdominal plate (12) to the back plate (13), wherein the at least one belt system (50) can be reversibly coupled to the abdominal plate (12) and/or the back plate (13), and wherein the at least one coupled belt system (50) can be retightened, **characterized in that**
the abdominal plate (12) and the back plate (13) are each articulated to the pelvic shell (11).

2. Shoulder abduction orthosis according to claim 1, wherein the abdominal plate (12) longitudinally extends at least to the middle of the abdomen, and wherein the back plate (13) longitudinally extends at least to the middle of the back.

3. Shoulder abduction orthosis according to any one of the preceding claims, wherein the abdominal plate (12) and the back plate (13) are configured to be bendable.

4. Shoulder abduction orthosis according to any one of the preceding claims, wherein the shoulder abduction orthosis (100) is a shoulder abduction orthosis without a shoulder belt.

5. Shoulder abduction orthosis according to any one of the preceding claims, wherein the at least one coupled belt system (50) can be retightened by means of at least one hook and loop fastener (51, 52).

6. Shoulder abduction orthosis according to any one of the preceding claims, wherein the at least one belt system (50) can be reversibly coupled to the abdominal plate (12) and/or the back plate (13), in each case by means of two rear-engaging elements (18a, 18b, 19a, 19b).

7. Shoulder abduction orthosis according to any one of the preceding claims, wherein the at least one belt system (50) has a width of at least 10 cm.

8. Shoulder abduction orthosis according to any one of the preceding claims, wherein the pelvic shell (11) has a pad (40) to suppress any slipping.

9. Shoulder abduction orthosis according to any one of the preceding claims, wherein the abdominal plate (12) and the back plate (13) are connected to the pelvic shell (11) in a fixable manner, in each case by means of a disk-shaped rotating joint (14, 15, 16, 17).

10. Shoulder abduction orthosis according to any one of the preceding claims, wherein the shoulder abduction orthosis (100) has an upper arm support (21) and a forearm support (21, 22).

## Revendications

1. Orthèse d'abduction d'épaule (100), comportant une monture pelvienne (10), et un support de bras (20), dans laquelle la monture pelvienne (10) comprend une coquille pelvienne (11), une plaque abdominale (12) et une plaque dorsale (13), le support de bras (20) étant relié avec la coquille pelvienne (10) au moyen d'un élément d'articulation à plusieurs axes (30), et la plaque abdominale (12) et la plaque dorsale (13) respectivement ayant une largeur d'au moins 10 cm,
dans laquelle la monture pelvienne (10) présente au moins un système des sangles (50) pour relier la plaque abdominale (12) avec la plaque dorsale (13), dans laquelle l'au moins un système des sangles (50) peut être couplé à la plaque abdominale (12) et/ou à la plaque dorsale (13) de manière réversible, et dans laquelle l'au moins un système des sangles (50) couplé peut être retendu, **caractérisée en ce que**
la plaque abdominale (12) et la plaque dorsale (13) sont respectivement articulées à la coquille pelvienne (11).

2. Orthèse d'abduction d'épaule selon la revendication 1, dans laquelle la plaque abdominale (12) s'étend en longueur au moins jusqu'au milieu de l'abdomen, et dans laquelle la plaque dorsale (13) s'étend en longueur au moins jusqu'au milieu du dos.

3. Orthèse d'abduction d'épaule selon l'une quelconque des revendications précédentes, dans laquelle la plaque abdominale (12) et la plaque dorsale (13) sont configurées de manière flexible.

4. Orthèse d'abduction d'épaule selon l'une quelconque des revendications précédentes, l'orthèse d'abduction d'épaule (100) étant une orthèse d'abduction d'épaule sans sangle d'épaule.

5. Orthèse d'abduction d'épaule selon l'une quelconque des revendications précédentes, dans laquelle l'au moins un système des sangles (50) couplé peut être retendu au moyen d'au moins une fermeture à crochets et boucles (51, 52).

6. Orthèse d'abduction d'épaule selon l'une quelconque des revendications précédentes, dans laquelle l'au moins un système des sangles (50) peut être couplé à la plaque abdominale (12) et/ou à la plaque dorsale (13) de manière réversible, respectivement au moyen de deux éléments à prise par l'arrière (18a, 18b, 19a, 19b).

7. Orthèse d'abduction d'épaule selon l'une quelconque des revendications précédentes, dans laquelle l'au moins un système des sangles (50) a une largeur d'au moins 10 cm.

8. Orthèse d'abduction d'épaule selon l'une quelconque des revendications précédentes, dans laquelle la coquille pelvienne (11) présente une pelote (40) antidérapante.

9. Orthèse d'abduction d'épaule selon l'une quelconque des revendications précédentes, dans laquelle la plaque abdominale (12) et la plaque dorsale (13) sont respectivement reliées de manière fixable à la coquille pelvienne (11) au moyen d'une articulation rotative (14, 15, 16, 17) sous forme de disque.

10. Orthèse d'abduction d'épaule selon l'une quelconque des revendications précédentes, l'orthèse d'abduction d'épaule (100) ayant un support d'arrière-bras (21) et un support d'avant-bras (21, 22).
